# EUROPEAN PATENT SPECIFICATION

(11) **EP 3 049 022 B1**
(45) Date of publication and mention of the grant of the patent: **07.06.2023**
(21) Application number: 13894862.5
(22) Date of filing: 24.09.2013
(51) Int. Cl.: A61F 2/16

(54) **ACCOMMODATING INTRAOCULAR LENS**
UNTERBRINGUNG VON INTRAOKULARLINSE
LENTILLE INTRAOCULAIRE D'ACCOMODATION

(43) Date of publication of application: 03.08.2016
(73) Proprietor: Cumming, J. Stuart, Laguna Beach, CA 92651 (US)
(72) Inventor: Cumming, J. Stuart, Laguna Beach, CA 92651 (US)
(74) Representative: J A Kemp LLP
(86) International application number: PCT/US2013/061452
(87) International publication number: WO 2015/047227

(56) References cited:
- US-A- 5 489 302
- US-A- 5 843 187
- US-A1- 2008 154 362
- US-A1- 2011 313 526
- US-B1- 6 197 059
- US-B1- 6 197 059
- US-B1- 6 387 126
- US-B2- 6 638 306

## Description

### BACKGROUND

### Field

The present disclosure is related to a single focus accommodating intraocular lens, various embodiments of which provide seamless vision from distance to near automatically by relaxation and constriction of ciliary muscle.

### Description of the Related Art

An intraocular lens (IOL) is a lens implanted into the eye, usually replacing a normal human lens that has been clouded over by a cataract, or can replace a normal human lens as a form of refractive surgery to change the eye's optical power.

Premium intraocular lenses (IOLs) implanted during cataract surgery are categorized three ways: accommodating, multifocal, and toric intraocular lenses.

The best visual acuity is achieved with the single focus accommodating lenses. The optic of these lenses moves forward and backward upon constriction and relaxation of the ciliary muscle. However, for reading in dim lighting conditions, or for small print, weak reading glasses are often necessary.

The multifocal lenses focus light on the retina at either two or three focal lengths. Thus, there is more than one image on the retina simultaneously. This creates problems since the amount of light in focus is divided between the multiple focal points, and contrast sensitivity is thereby reduced, making vision at all distances difficult in dim lighting. In addition, there are severe problems when driving at night when the pupil is dilated. Many patients experience severe glare and halos and many have had to have the multifocal lenses explanted and replaced with a single vision standard lens, because of this problem. However, the near vision with the multifocal lenses is superior to that of the current accommodating lens.

The toric lenses correct the eyes that have significant astigmatism.

The currently marketed plate accommodating intraocular lenses provide excellent distance and intermediate vision but sometimes can require weak, +1.00, reading glasses for prolonged reading, for seeing small print, or reading in dim lighting conditions.

Furthermore, it is important for intraocular lenses to have a consistent location along the axis of the eye to provide good uncorrected distance vision and to center in the middle of the vertical meridian of the eye. Without excellent uncorrected distance vision there is no point in implanting lenses designed to give seamless vision from far to near.

The original intraocular lens consisted of a single optic. These lenses frequently decentered and dislocated and it was discovered that there was a need to center and fixate the lens optic in the vertical meridian of the eye.

Attachments to the optic that center and fixate the lens within the capsular bag are called haptics. Traditionally, haptics comprise multiple flexible loops of various designs, J loops, C loops, closed loops, and flexible radial arms. Recently, traditional haptics have been replaced in some lens designs with oblong, flat flexible plates, called plate haptics. These plate haptics usually made from silicone, are solid, flat, flexible and between 3.0 and 6.0 mm in width, 0.20 to 0.75 mm thick, and may have tapered, rounded or parallel sides. Plate haptics often have flexible loops or fingers that help center and fixate the lens within the capsular bag. These flexible fingers extend beyond the distal or outer end of the plate haptics and slightly beyond the diameter of the capsular bag and are designed to flex centrally to center and fixate the lens and its optic within the capsular bag.

An accommodating IOL (AIOL) permits refocusing of the eye by means of movement along the optical axis in response to the constriction or relaxation of ciliary muscles. Near vision results from a forward movement of the optic upon constriction of the ciliary muscle which increases the pressure in the posterior part of the eye with a simultaneous decrease in pressure in the anterior part of the eye. Distance vision results from the reverse pressure change that takes place upon relaxation of the ciliary muscle and the resultant backwards movement of the lens. The movement of the optic enables the patient implanted with the lens to automatically change their vision between far, intermediate and near.

AIOLs are known to include opposing haptics positioned on either side of a lens optic. Once a patient's cataract is removed, e.g. by phacoemulsification, the AIOL is placed into the empty capsular bag. The haptics help to center the AIOL and fixate it within the capsular bag by fibrosis. Such AIOLs are described in U.S. Patent No. 5,674,282, U.S. Patent No. 5,476,514, and U.S. Patent No. 5,496,366, to Cumming.

Moreover, although current AIOLs provide patients with significantly restored distance and intermediate vision, adequate near vision is commonly lacking - often requiring that patients use weak reading glasses to enhance near vision.

US 6,197,059 discloses an accommodating intraocular lens to be implanted within the natural capsular bag of a human eye from which the natural lens matrix has been removed through an anterior capsule opening in the bag circumferentially surrounded by an anterior capsular remnant. During a postoperative healing period following surgery, the anterior capsular remnant fuses to the posterior capsule of the bag by fibrosis about haptics on the implanted lens while the ciliary muscle is maintained in its relaxed state by a cycloplegic to prevent discloation of the lens, and the lens is deflected rearwardly by the fibrosing anterior capsular remnant to a distant vision position against the elastic posterior capsule of the bag in which the posterior capsule is stretched rearwardly. After fibrosis is complete, natural brain-induced contraction and relaxation of the ciliary muscle relaxes and stretches the fibrosed anterior remnant and increases and reduces vitreous pressure in the eye to effect vision accommodation by the remnant, the posterior capsule, and vitreous pressure.

US 2011/313526A discloses a plate haptic for an accommodating intraocular lens. The plate haptic has a haptic body that is substantially rigid in a longitudinal direction and substantially flexible in a transverse direction. A chassis is integral to the haptic body. The chassis causes the haptic body to be substantially more rigid in a longitudinal direction than in a transverse direction.

### SUMMARY

An accommodating intraocular lens according to the present invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

In various embodiments, an accommodating intraocular lens comprises a lens optic coupled to at least one haptic and a torsion bar positioned between the lens optic and the at least one haptic such that the torsion bar facilitates accommodation by deforming in response to a vitreous pressure change.

The connecting portion unlike a flat hinge flexing mechanism or a flat thin stretching hinge may have a cross section that can be round, oval, square or any variation of these. The connecting portion can have a length of between about 0.1 to 1.5 mm, in various embodiments between about 0.5 and 1.0 mm, or between about 0.1 mm to 1.0 mm. The connecting portion can connect tangentially to the optic in a transverse direction across the lens body, or connect in a radial or perpendicular manner, to function by stretching, thinning, rotating, or any combination of these.

Certain aspects of this disclosure are directed toward an accommodating intraocular lens. The intraocular lens can include a lens optic having a diameter, at least one haptic coupled to the lens optic, and at least one connecting bar connecting the lens optic and the at least one haptic. The connecting bar can facilitate accommodation by deforming in response to a vitreous pressure change. The connecting bar can have a width and thickness less than 30% the diameter of the optic.

Certain aspects of this disclosure are directed toward an accommodating intraocular lens having a lens optic coupled to at least one haptic. The at least one haptic can be primarily disposed on opposite first and second sides of the lens optic. The first side, the lens optic, and the second side can be arranged along a longitudinal axis of the accommodating intraocular lens. The at least one connecting bar can connect the lens optic and the at least one haptic. The at least one connecting bar can have a length disposed substantially orthogonal to said longitudinal axis of said accommodating intraocular lens.

Certain aspects of this disclosure are directed toward an accommodating intraocular lens having a lens optic coupled to at least one haptic. The at least one haptic can be primarily disposed on opposite first and second sides of the lens optic. The first side, the lens optic, and the second side can be arranged along a longitudinal axis of the accommodating intraocular lens. The at least one connecting bar can connect the lens optic and the at least one haptic. The at least one connecting bar can have a length disposed substantially parallel to said longitudinal axis of said accommodating intraocular lens.

Certain aspects of this disclosure are directed toward an accommodating intraocular lens having a lens optic coupled to at least one haptic. The at least one haptic can be primarily disposed on opposite first and second sides of the lens optic. The first side, the lens optic, and the second side can be arranged along a longitudinal axis of the accommodating intraocular lens. The at least one connecting bar can connect the lens optic and the at least one haptic. The at least one connecting bar can have a length disposed at an angle within about 30 degrees (e.g., within about 10 degrees, about 15 degrees, within about 20 degrees, or about 25 degrees) from said longitudinal axis of said accommodating intraocular lens.

Certain aspects of this disclosure are directed toward an accommodating intraocular lens having a lens optic coupled to at least one haptic. The at least one haptic can be primarily disposed on opposite first and second sides of the lens optic. The first side, the lens optic, and the second side can be arranged along a longitudinal axis of the accommodating intraocular lens. The at least one connecting bar can connect the lens optic and the at least one haptic. The at least one connecting bar can have a length disposed at an angle within about 30 degrees (e.g., at least about 10 degrees, about 15 degrees, about 20 degrees, or about 25 degrees) from an axis orthogonal to said longitudinal axis of said accommodating intraocular lens (i.e., a transverse axis of said accommodating intraocular lens).

In any of the above mentioned aspects, the intraocular lens includes an optic appendage attached to said lens optic. The connecting bar can connect the optic appendage and the at least one haptic.

In any of the above mentioned aspects, the connecting bar can have a length between about 0.1 and 2.0 mm.

In any of the above mentioned aspects, the connecting bar can have a circular cross-section orthogonal to the length of the bar. In other aspects, the connecting bar can have an ovular cross section orthogonal to the length of the bar. In yet other aspects, the connecting bar can have a square cross-section orthogonal to the length of the bar.

In any of the above mentioned aspects, the connecting bar can be configured to elongate when subjected to pressure changes. In certain aspects, the connecting bar can be configured to thin when subjected to pressure changes. In certain aspects, the connecting bar can be configured to rotate when subjected to pressure changes.

In any of the above mentioned aspects, the connecting bar can include the same material as the haptic. In other aspects, the connecting bar includes a different material than the haptic.

In any of the above mentioned aspects, the intraocular lens can include an elongate slot partially traversing the haptic in the transverse direction of said intraocular lens. In certain aspects, the slot can be between 0.1 to 0.5 mm in height and 2 to 5 mm in length. In certain aspects, the connecting bar can be configured to permit forward movement of the lens optic by stretching more than bending in response posterior pressure comparable to the vitreous pressure of an accommodating eye.

In any of the above mentioned aspects, the intraocular lens may not include a hinging feature between the lens optic and the haptic.

Other features and advantages of the present disclosure will become apparent from the following more detailed description, taken in conjunction with the accompanying drawings, which illustrate, by way of example, the principles of the presently described apparatus and method of its use.

### BRIEF DESCRIPTION OF THE SEVERAL VIEWS OF THE DRAWING(S)

Figure 1 illustrates a top plan view of an AIOL according to at least one embodiment of the present disclosure with transversely directed torsion bars connecting the optic to the haptics.
Figure 2 illustrates a portion of an AIOL according to another embodiment of the present disclosure with generally longitudinally directed connector bars connecting the optic to the haptics.

### DETAILED DESCRIPTION

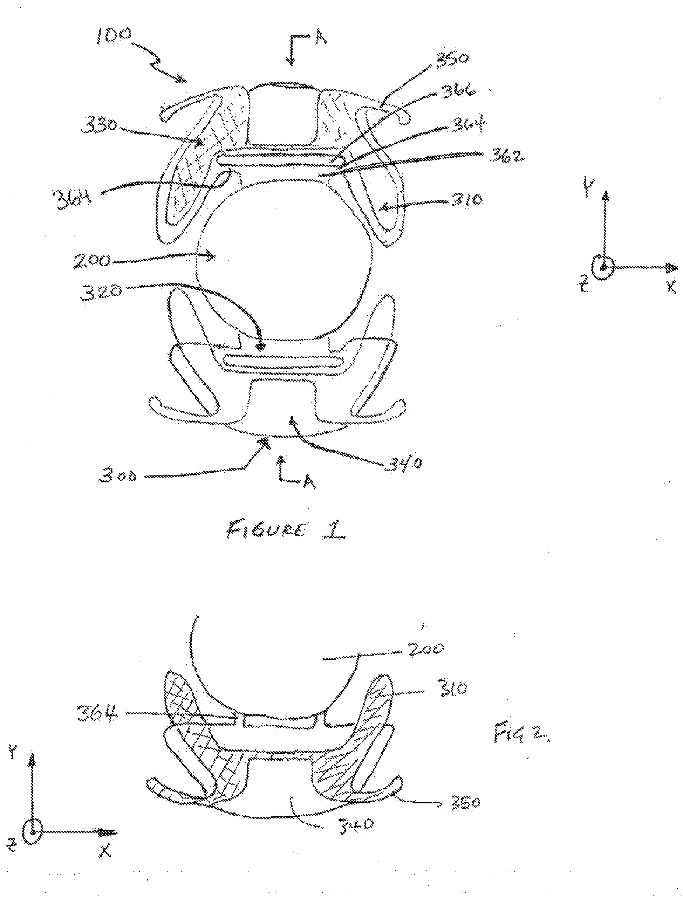

FIG. 1 illustrates accommodating intraocular lens (AIOL) 100 comprising an optic 200 a short appendage 362 attached to the optic and coupled to at least one haptic 300 as well as connection bars 364 connecting the optic to the at least one haptic via the appendage 362.

The AIOL 100 is placed into the capsular bag of a patient's eye during cataract surgery involving known techniques such as, for example, phacoemulsification. The lens is centered so that the optical axis of the lens coincides with that of the patient's eye. The haptics 300 contact the capsular bag and the natural fibrosis of the tissue secures the haptics 300, and consequently the AIOL 100, in place.

The optic 200 is preferably a single focus optic that gathers the incoming light and focuses it on the retina of the patient to affect vision. The optic 200 may be biconvex, refractive, diffractive, plano-convex, Fresnel, spheric, aspheric, toric, or of any other type that is preferably substantially single focus. In order to permit the optic **200** to be inserted into the eye through a small incision, the optic **200** is preferably made of a flexible optical material, such as, for example, silicone, acrylic, hydrogel, or other flexible optical material now known or hereafter developed.

The at least one haptic **300** comprises a first end **320** opposite a second end **340.** The first end **320** of the haptic **300** can be proximal the optic **200** while the second end is distal to the optic. The first end **320** is flexibly coupled to a periphery of the optic **200** or short appendage **362.** In at least one embodiment, the AIOL **100** comprises opposing haptics positioned linearly along a longitudinal axis **A** of the AIOL **100** (e.g., parallel to the y-axis).

The haptic body may be substantially flexible in the transverse direction (e.g., parallel to the x-axis) and substantially rigid in the longitudinal direction (e.g., parallel to the y-axis) to enable the AIOL **100** to be folded and inserted into the eye via a small incision. While the plate haptic is substantially rigid, the flexibility of the connecting bar may promote vaulting. In various embodiments, the haptic **300** is constructed of the same or similar flexible material as the optic, including, but not limited to: silicone, hydrogel, acrylic, or similar material.

The plate haptic **300** may comprise opposing lateral paddles **310.** The paddles **310** and projecting fingers **350** operable to engage, fixate, and center the AIOL in the capsular bag. In certain aspects, the paddles **310** can include a different, more rigid material than the plate haptics **300.** For example, the paddles **310** can be made of polyimide, prolene, or titanium. The paddles may be useful for dipping into the vitreous cavity to further increase the pressure. The paddles also help stabilize the lens to prevent the AIOL from tilting. The paddles can also prevent anterior dislocation of one of the two plate haptics. Such exemplary haptics **300** and paddles **310** are discussed in U.S. Patent Publication 2011-0313519 A1, published December 22, 2011 (U.S. Patent Nos. 13/017,189); U.S. Patent Publication 2011-0313524 A1, published December 22, 2011 (U.S. Patent Nos. 13/092,359); U.S. Patent Publication 2011-0313525 A1, published December 22, 2011 (U.S. Patent Nos. 13/111,599); and U.S. Patent Publication 2011-0313526 A1, published December 22, 2011 (U.S. Patent Nos. 13/155,327).

The paddles **310** and/or the plate haptics **300** together can surround at least a portion of the optic or surround a majority of the optic. The paddles **310** and/or the plate haptics **300** together can surround between about 100 to 350 degrees of the optic, for example, between about 100 and 200 degrees, between about 150 degrees and 250 degrees, between about 200 degrees and about 300 degrees, or between about 250 degrees and 350 degrees. The paddles **310** together extend along the sides of the optic and more particularly about 50%, 60%, 70%, 80% or more of the length of the optic. In various embodiments, the paddles **310** extend along the optic **200** in the lateral direction (parallel to the x-axis) at least about 10% the diameter of the optic. In certain aspects, the paddles can include curved edges, for example, as shown in the figures, the paddles can curve inward to at least partially conform to the shape of the optic. In certain aspects, a length of the paddle can be at least two times, at least three times, or at least four times longer than a width of the paddle. In certain aspects, an end of the paddle closest to the optic can be tapered.

A frame **330** may be embedded within the haptic **300.** The frame **330** may be formed of polyimide, prolene, polymethylmethanylate (PMMA), titanium, or similar material. The frame **330** may be a meshwork or lattice, designed and shaped in a manner to make the plate haptics rigid longitudinally but flexible transversely. Such exemplary frames **330** are discussed in U.S. Patent Publication 2011-0313519 A1, published December 22, 2011 (U.S. Patent Nos. 13/017,189); U.S. Patent Publication 2011-0313524 A1, published December 22, 2011 (U.S. Patent Nos. 13/092,359); U.S. Patent Publication 2011-0313525 A1, published December 22, 2011 (U.S. Patent Nos. 13/111,599); and U.S. Patent Publication 2011-0313526 A1, published December 22, 2011 (U.S. Patent Nos. 13/155, 327).

The haptic **300** may further comprise projections **350,** or fingers, extending from the second end (distal to the optic **200)** to engage the capsular bag and secure and center the AIOL thereto. The projections **350** may be homogeneous with the frame and may be made of either polyimide, PMMA, acrylic or any other inert material. Such exemplary projections 350 are discussed in U.S. Patent Publication 2011-0313519 A1, published December 22, 2011 (U.S. Patent Nos. 13/017,189); U.S. Patent Publication 2011-0313524 A1, published December 22, 2011 (U.S. Patent Nos. 13/092,359); U.S. Patent Publication 2011-0313525 A1, published December 22, 2011 (U.S. Patent Nos. 13/111,599); and U.S. Patent Publication 2011-0313526 A1, published December 22, 2011 (U.S. Patent Nos. 13/155,327).

The first end **320** of the haptic **300** (e.g., proximal to the optic **200)** may be coupled to the optic or short appendage of the optic **362** via one or more connecting portions. The connecting bars **364** may operate to permit contraction of the ciliary muscles to cause an end-to-end compression of opposing haptics with an increase in vitreous pressure, with resultant movement of the optic being substantially forward. The connecting portions, bars, or members, **364** shown in FIG. 1 are capable of stretching, bending, and/or potentially rotating when subjected to posterior pressure. Depending on the configuration of the connecting portions **364,** the connecting portions **364** may stretch along the length thereof, which may be at least in part along a direction substantially parallel to the longitudinal axis of the AIOL and/or may be at least partly along an axis that is substantially perpendicular to the longitudinal axis of the AIOL or along the transverse direction (e.g., parallel to x-axis and/or z-axis). Further, the connecting portions **364** may twist or rotate about an axis along the length of the connecting portion, which may be about an axis that is substantially perpendicular to the longitudinal axis of the AIOL (e.g. about an axis parallel to z-axis and/or x-axis) and/or may be about an axis that is substantially parallel to the longitudinal axis of the AIOL (e.g. about an axis parallel to y-axis) in some cases. In some embodiments, each of the connecting portions **364** can be positioned adjacent the optic periphery and flexibly coupled thereto. The connecting portions **364** can be oriented such that their length extends along an axis substantially parallel to a transverse axis of the AIOL **100** (e.g. along an axis parallel to the x-axis) as shown in FIG. 1 or substantially parallel to a longitudinal axis of the AIOL **100** as shown in FIG. 2.

With continued reference to Figure 1, as disclosed above, one or more connecting portions **364** can be oriented along an axis substantially parallel to a transverse axis of the AIOL **100** (e.g. parallel to the x-axis). The AIOL **100** can include two connecting portions **364** for securing each haptic **300** to the short appendage **362** and optic **200.** The connecting portions **364** can connect the haptic **300** to the short appendage **362** such that an elongate slot **366** is formed between the short appendage **362** and the haptic **300.** An elongate axis of the elongate slot **366** can be substantially perpendicular to the longitudinal axis of the AIOL through line A-A (e.g. perpendicular to the y-axis) in some embodiments. Although not shown, the connecting portions 364 may be oriented along an axis that is less than or equal to about 5°, 10°, 15° 20°, 25°, 30°, 35°, 40°, or 45° from the transverse axis of the AIOL (e.g., axis parallel to x-axis).

The elongate slot **366** is an aperture formed in the haptic **300** that extends laterally and parallel to the transverse direction (e.g. parallel to the x-axis). Preferably, the slot **366** comprises an oval shape, but all shapes are specifically contemplated. As mentioned above, the slot **366** is adjacent the connecting portions distal to the optic **200.** Preferably, the slot dimensions ranging from 0.1 to 0.5 mm in height and 2.0 to 5.0 mm in length.

As shown in Figure 2, one or more connecting portions or connecting bar **364** can be oriented or extend along an axis that is substantially parallel to a longitudinal axis of the AIOL **100** (e.g. along an axis parallel to the y-axis). In some embodiments, the AIOL **100** can include two connecting portions or bars **364** for securing each haptic **300** to the optic **200** although more or less are possible. For example, although not shown, each haptic 300 can be connected to the optic 200 by a single connecting portion 364. Reducing the number of connecting portion 364 can be beneficial to decrease the force necessary to move the optic relative to the haptics. Although not shown, the connecting portions 364 may be oriented along an axis that is less than or equal to about 5°, 10°, 15° 20°, 25°, 30°, 35°, 40°, or 45° from the longitudinal axis of the AIOL (e.g., axis parallel to y-axis).

The connecting portions **364** can connect the haptic **300** to the optic **200** such that an elongate slot is formed between the optic **200** and the haptic **300.** An elongate axis of the elongate slot can be substantially perpendicular to the longitudinal axis of the AIOL through line A-A (e.g. parallel to the x-axis).

The connecting portions **364** couples the optic **200** to the haptic **300** and is preferably of the same material as the optic. The connecting portions **364** assist in accommodation in that they decrease the resistance to the pressure that pushes the optic forward. In various embodiments, such connecting portions **364** are not hinges in the sense that they primarily stretch and do not bend like a hinge when the optic **200** is vaulted forward because of an increase in vitreous pressure during accommodation. Likewise, in various embodiments, the connecting portions **364** may not include a hinge feature, for example, between the optic **200** or appendage **362** and the haptic **300** about which portions of the connecting portion bend like a hinge. Example hinges or straps with hinge features are described in U.S. Patent Publication 2011-0313519 A1, published December 22, 2011 (U.S. Patent Nos. 13/017,189); U.S. Patent Publication 2011-0313524 A1, published December 22, 2011 (U.S. Patent Nos. 13/092,359); U.S. Patent Publication 2011-0313525 A1, published December 22, 2011 (U.S. Patent Nos. 13/111,599); and U.S. Patent Publication 2011-0313526 A1, published December 22, 2011 (U.S. Patent Nos. 13/155,327).

The connecting portions or bars **364** can extend lengthwise laterally (as in FIG. 1) or longitudinally (as in FIG. 2) between opposing paddles of the haptic along an axis either substantially parallel or substantially perpendicular to the longitudinal axis of the AIOL through line A-A. Moreover, the connecting portions are in various embodiments integral to the haptic **300** at each of the lateral ends (as shown in FIG. 1) or at each of the end distal to the optic **200** (as shown in FIG. 2), and are preferably of the same flexible material as the haptic. However, the connecting portions **364** may also be of a different material than the haptic **300,** for example, such that the connecting portions are substantially more flexible than the haptic. In at least one embodiment, the connecting portions **364** may be from 0.1 to 1.5 mm in length. Preferably, the connecting portion **364** has a square or circular cross-section, but other cross-sectional solid shapes are specifically contemplated. Accordingly, in various embodiments, the cross-section has a width and height that are substantially equal and may be smaller than the length of the connecting portion or bar **364.** For example, the width of the cross-section perpendicular to the length of the connecting portion or bar **364** may be less than 1.5, 1.4, 1.3, 1.2, or 1.0 times the height of the cross-section or may be smaller and possible larger in some embodiments. Similarly, the width of the cross-section perpendicular to the length of the connecting portion or bar **364** may be less than 2.0, 1.5, 1.2, or 1.0 times the length of connecting portion or bar or may be smaller and possible larger in some embodiments.

On insertion into the eye, the optic **200** and haptics **300** may be vaulted posteriorly. The haptics may move centrally and posteriorly in response to ciliary muscle contraction, i.e. end-to-end compression. Such movement increases the vitreous pressure, causing the optic to vault forward by stretching and potentially thinning of the connecting portion **364.** This increase in pressure is further facilitated by the paddles dipping posteriorly into the vitreous cavity. This effect is facilitated by the stretching, and potentially thinning, bending, and/or rotation of the connecting portions in response to the increase in pressure. Relaxation of the ciliary muscle increases the diameter of the ciliary muscle and reduces the vitreous cavity pressure with a concomitant increase in pressure in the anterior part of the eye such that the optic **200** moves posteriorly to the distant vision position.

In at least one embodiment, the longitudinal length of the AIOL (e.g., from distal end to distal end, wherein the distal end is distal with respect to the optic **200**) may be between approximately 9.0-11.0 mm, with the diameter as measured from the tips of the lateral finger projections with respect to the optic being between approximately 11.5-12.0 mm. The haptics **300** are in various embodiments between 3.0-6.0 mm wide and 0.20-0.75 mm thick, while the optic **200** may be approximately 5.0 mm in diameter and 1.0 mm thick at its center.

In various embodiments the connecting portions or connecting bars **364** are thin and experience more thinning when stretched. Without stretching, for example, the connecting portions or connecting bars **364** may be thin with respect to the optic **200** and the lens **100.** In certain embodiments, for example as shown in Fig. 2, the connecting portion or connecting bar **364** may have a width in the transverse direction (e.g., parallel to the x-axis) less than 50%, 40%, 30%, 20%, 10%, 5% or less of the width or diameter of the optic 20. Similarly, the connecting portions or connecting bars **364** may have a thickness orthogonal to the transverse and longitudinal directions (e.g., parallel to the z-axis) that is less than 50%, 40%, 30%, 20%, 10%, 5% or less of the diameter of the width or diameter of the optic 20. The width and thicknesses may be greater than 0.5% or 1% of width or diameter of the optic 20 in some embodiments.

The dimensions described above allow for adequate stretching. Additionally, such widths may reduce the incidence of tearing when inserting the AIOL into the small opening made in the sclera or cornea of the eye. The AIOL is rolled to facilitate insertion and the resultant twisting can introduce shear forces on wider connections, which may cause tearing. Having narrow widths reduces the shear force and permits twisting without tearing.

The connecting portions or bars **364** such as shown in FIGS. 1 and 2 being thin permit stretching. In various embodiments, the connecting portions or bars **364** stretch and thereby increase their length by at least 0.5, 1.0, 1.5, or 2.0 times of their length or more. Accordingly, in some embodiments such as the configuration shown in FIG. 2 and possibly the configuration shown in FIG. 1, the connecting portions or bars **364** operate to permit vaulting forward of the optic during accommodation more by stretching than by bending. Likewise, in various embodiments, the connecting portions or bars **364** are configured to primarily stretch rather than bend (e.g., like a hinge) when the lens is exposed to posterior pressure akin to the increase pressure in the posterior part of the eye with an accommodating eye as well as a simultaneous anterior pressure akin to the simultaneous decrease in pressure in the anterior part of the eye during accommodation.

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated.

## Claims

1. An accommodating intraocular lens (100) comprising:
a lens optic (200) coupled to two haptics (300), said two haptics disposed on opposite first and second sides of the lens optic, wherein the first side, the lens optic (200), and the second side are arranged along a longitudinal axis (A-A) of said intraocular lens;
a plurality of connecting bars (364) connecting the lens optic (200) to the two haptics (300), the connecting bars (364) having a length disposed orthogonal to said longitudinal axis (A-A) of said intraocular lens (100);
an optic appendage (362) attached to said lens optic (200), wherein the connecting bar (364) connects the optic appendage (362) and said haptics (300), **characterised in that** the accommodating intraocular lens is provided with an elongate slot (366) formed between the optic appendage (362) and said haptics (300), the elongate slot (366) partially traversing said one of the two haptics (300) in a transverse direction orthogonal to the longitudinal axis;
wherein the connecting bars (364) are flexibly coupled to the lens optic periphery (200) so that the connecting bars (364) can rotate about an axis extending along their length when the intraocular lens (100) is subjected to a pressure change.

2. The intraocular lens (100) of Claim 1, wherein:
the length of the connecting bars (364) is between 0.1 and 2.0 mm.

3. The intraocular lens (100) of any one of Claims 1 to 2, wherein the elongate slot (366) is between 0.1 to 0.5 mm in height and 2 to 5 mm in length.

## Patentansprüche

1. Akkommodierende Intraokularlinse (100), die Folgendes umfasst:
eine Linsenoptik (200), die mit zwei Haptiken (300) gekoppelt ist, wobei die beiden Haptiken auf gegenüberliegenden ersten und zweiten Seiten der Linsenoptik angeordnet sind, wobei die erste Seite, die Linsenoptik (200) und die zweite Seite entlang einer Längsachse (A-A) der Intraokularlinse ausgelegt sind;
eine Vielzahl von Verbindungsstäben (364), die die Linsenoptik (200) mit den beiden Haptiken (300) verbinden, wobei die Verbindungsstäbe (364) eine Länge aufweisen, die orthogonal zu der Längsachse (A-A) der Intraokularlinse (100) angeordnet ist;
einen Optikfortsatz (362), der an der Linsenoptik (200) angebracht ist, wobei der Verbindungsstab (364) den Optikfortsatz (362) und die Haptiken (300) verbindet, **dadurch gekennzeichnet, dass** die akkommodierende Intraokularlinse mit einem länglichen Schlitz (366) versehen ist, der zwischen dem Optikfortsatz (362) und der Haptik (300) gebildet ist, wobei der längliche Schlitz (366) die eine der beiden Haptiken (300) in einer Querrichtung orthogonal zur Längsachse teilweise durchquert;
wobei die Verbindungsstäbe (364) flexibel mit dem Linsenoptikumfang (200) gekoppelt sind, so dass die Verbindungsstäbe (364) um eine Achse rotieren können, die sich entlang ihrer Länge erstreckt, wenn die Intraokularlinse (100) einer Druckänderung unterworfen wird.

2. Intraokularlinse (100) nach Anspruch 1, wobei:
die Länge der Verbindungsstäbe (364) zwischen 0,1 und 2,0 mm beträgt.

3. Intraokularlinse (100) nach einem der Ansprüche 1 bis 2, wobei der längliche Schlitz (366) eine Höhe von 0,1 bis 0,5 mm und eine Länge von 2 bis 5 mm aufweist.

## Revendications

1. Lentille intraoculaire accommodative (100), comprenant :
une optique de lentille (200) couplée à deux haptiques (300), lesdites deux haptiques étant disposées sur des premier et second côtés opposés de l'optique de lentille, dans laquelle le premier côté, l'optique de lentille (200), et le second côté sont agencés le long d'un axe longitudinal (A-A) de ladite lentille intraoculaire ;
une pluralité de barres de liaison (364) reliant l'optique de lentille (200) aux deux haptiques (300), les barres de liaison (364) ayant une longueur disposée orthogonalement audit axe longitudinal (A-A) de ladite lentille intraoculaire (100) ;
un appendice d'optique (362) attaché à ladite optique de lentille (200), dans laquelle la barre de liaison (364) relie l'appendice d'optique (362) et lesdites haptiques (300), **caractérisée en ce que** la lentille intraoculaire accommodative est pourvue d'une fente allongée (366) formée entre l'appendice d'optique (362) et lesdites haptiques (300), la fente allongée (366) traversant partiellement ladite une des deux haptiques (300) dans une direction transversale orthogonale à l'axe longitudinal ;
dans laquelle les barres de liaison (364) sont flexiblement couplées à la périphérie de l'optique de lentille (200) pour que les barres de liaison (364) puissent tourner autour d'un axe s'étendant le long de leur longueur lorsque la lentille intraoculaire (100) est soumise à un changement de pression.

2. Lentille intraoculaire (100) selon la revendication 1, dans laquelle :
la longueur des barres de liaison (364) est entre 0,1 et 2,0 mm.

3. Lentille intraoculaire (100) selon l'une quelconque des revendications 1 et 2, dans laquelle la fente allongée (366) fait entre 0,1 et 0,5 mm de haut et 2 et 5 mm de long.
